# EUROPEAN PATENT SPECIFICATION

(11) **EP 2 037 949 B1**
(45) Date of publication and mention of the grant of the patent: **29.02.2012**
(21) Application number: 07721839.4
(22) Date of filing: 12.06.2007
(51) Int. Cl.: A61K 35/14, A61K 38/17, B01D 61/00

(54) **A METHOD OF MANUFACTURING A TRANSFER FACTOR MEDICAMENT**
METHODE ZUR HERSTELLUNG EINES TRANSFER FAKTOR MEDIKAMENTS
MÉTHODE POUR LA PRODUCTION DE MÉDICAMENT FACTEUR DE TRANSFERT

(30) Priority: 13.06.2006 CZ 20060377
(43) Date of publication of application: 25.03.2009
(73) Proprietor: Sevapharma A.S., 102 19 Praha 10 (CZ)
(72) Inventor: PEKÁREK Jan, CZ-160 00 Praha 6 (CZ)
(74) Representative: Danek, Vilém
(86) International application number: PCT/CZ2007/000050
(87) International publication number: WO 2007/143957

(56) References cited:
- EP-A- 0 101 200
- CN-A- 1 093 907
- DE-A1- 3 244 607
- DATABASE WPI Week 200608 Derwent Publications Ltd., London, GB; AN 2006-068560 XP002471168 & CN 1 654 477 A (BEIJING CENTURY ZHAOBAO TRANSFER FACTOR RES CENT) 17 August 2005 (2005-08-17)
- DATABASE WPI Week 200547 Derwent Publications Ltd., London, GB; AN 2005-459418 XP002471178 & CN 1 589 897 A (UNIV XIBEI SCI & TECHNOLOGY AGRIC & FORE) 9 March 2005 (2005-03-09)

## Description

### Technical Field

Technical solution covering a pharmaceutical manufacturing of a medicament called "transfer factor".

### Background Art

In fifties and sixties of 20^{th} century, a mechanism of cellular immunity has been described (immunity mediated by cells) as well as the mechanism of this type of immunity transportation - transfer factor. The transfer factor represents itself a mixture of substances with relative molecular weight less than 10 000, that do not contain transplantation antigens. This transfer factor can mediate also interspecies transfer of cellular immunity and this phenomenon can be used in therapy of diseases linked to immune system, especially to its lymphocyte part, particularly T-lymphocytes.

It is well known that transfer factor can be obtained by means of different methods, dialysis or ultrafiltration being the most frequent of them. "Transfer Factor" medicament is a dialysate or ultrafiltrate of peripheral leucocytes mixture obtained from healthy donors that have been tested and found non-reactive against HIV 1+2, HCV, HBsAg and lues. The proper examinations anti-HIV 1+2, anti-HCV and anti HBsAg are performed in every blood sample at least twice. Each individual donor's blood is examined once, next it is blood obtained from 10 different donors. These examinations are performed by a method that is currently accepted by local legal authority. The examined sample must not show any reactivity that could be taken for positive in used method. Syphilis examination is always performed on every sampling by at least one test, ideally by combined non-specific (cardiolipin) and specific (treponema) test. Tested sample must not show reactivity considered positive.

### Summary of the invention

The present disclosure, comprising the present invention provides a method of manufacturing "transfer factor" medicament having therapeutic effects based on donor immune system normalizing and containing a complex and a combination of biologically active low molecular substances having MW under 10000 with at least 200 biological activities, including transfer factors in donors blood, as are precursors of maturation and activation of cells, manufactured according to claim 1.

Molecular profile of transfer factor is determined by highly effective (high pressure) liquid chromatography and moderate liquid chromatography providing that: isocratic flow, column suitable for gel chromatography with optimal particle size approx. 5 µm and pores size aprox. 12,5 nm. Suitable molecular weight standards, e.g. bovine albumin, ribonuclease, p-aminobenzoic acid.

Transfer factor therapeutic effect of is based especially on donor immune system normalizing. Clinical effect of transfer factor comes from the group of substances contained in the medicament, the way of its manufacturing and use; not only as a matter of pure biological compounds. The drug takes advantage of both non-specific pharmacodynamic features of product and possible particular antigen specificity (a specific transfer factor). The use of transfer factor represents itself an example of so-called "information therapy" based on the selected signal inserting into body that is amplified inside the body changing its activity, i.e. the way of answer to particular disease.

As a mobile phase, phosphate buffer is used, with pH 6,5 - 7,5, permanent temperature 25°C or 30°C (± 2°C ), wave length 260 nm, length of run approx. 20 - 30 min., used sample volume approx. 20 µl.

Both qualitative and quantitative assessments of transfer factor are based on the determination of simple fractions approximate molecular weigh using retention times of particular areas peaks. Drug description leads to the conclusion that this analysis must not prove the share of parts having relative molecular weight more than 10 000 higher than 1 %. As an advantage, the remedy can contain the potion of parts with relative molecular weight approx. 2000 - 6000 corresponding to 65 - 95 % and that of relative molecular weight approx. 1000 - 2000 being 10 - 30 % or higher. Another advantage appears if remedy contains less than 10% of compounds with relative molecular weight less than approx. 1000. The contents expressed as a percentage is always understood to mean the volume value.

The remedy is manufactured in a lyophilised form. Lyophilised transfer factor is slightly yellowish - white substance with consistency from powder to spongy. Having been dissolved in water, a pure slightly yellowish or yellowish fluid appears. One dose of product, intended for one therapeutic administration, represents the amount of biologically active substances derived from approx. 200 millions of leucocytes. The remedy does not contain any antimicrobial.preservatives. Drug sterility is based only by its aseptic manufacturing.

The manufacturing procedure involves following steps:
a) The adjustment of leucocytes homogenate
b) Dialysis or ultrafiltration
c) Concentration by means of lyophylisation
d) The preparation of raw medical solution
e) Interoperating tests
f) Homogenisation
g) Pre-filtration
h) Ultrafiltration
i) Sterilisation filtration
j) Termic pasteurisation
k) Filling in ampoules and fulfilment
l) Lyophylisation

### a) Leucocytes homogenate preparation:

Leucocytes concentrates obtained from particular donors are transported and stored in frozen status. As soon as defrosted in +37 °C, the raw material originating from separate donors is connected to get a bigger volume. The procedure must be performed in manufacturing areas with defined environmental purity. As a following step, all the membrane components are broken by means of repeated freezing and defrosting, in some case by ultrasound cell destruction or by both the approaches combined.

- The preparation of homogenate using method of freezing - defrosting:
Freezing is performed in bath of denatured alcohol in the temperature at least -35 °C using rotation freezing machinery.
Defrosting is performed in a water bath of not more than + 37 °C. The complete cycle of freezing - defrosting is repeated 6 times. Following the 6^{th} cycle, homogenate in original bottles is given to freezing box set up to at least -17 °C till the next procedure.

- The preparation of homogenate using method of ultrasound:

Following defrosting, a leucocytes homogenate is obtained by ultrasound destruction of cells in 20 kHz for 10 minutes in the temperature of + 2 °C - + 8 °C. Following this disintegration, the homogenate obtained is stored in freezing box in less than 17°C till the next defrosting.

- The preparation of homogenate using combined method:

An alternative procedure of leucocytes homogenisation is a combined method. The cycle of freezing and defrosting is repeated 5x with successive 20 kHz ultrasound disintegration,for 3 minutes in temperature + 2 °C - + 8 °C. Following this disintegration, the homogenate is put into freezing box in not more than -17°C till the next procedure.

All the temperatures used in this production step must not vary with amplitude higher than 30°C up and down and variation should not be longer than several minutes.

During the preparation of leucocytes homogenate that originates by mixture of separate leucocytes concentrates (obtained from particular donors in transfusion units), concentrates of leucocytes are mixed together into homogenate following their defrosting (see the above described manufacturing description). The substance is then destroyed, biologically inert and no interaction between substances obtained from different donors occur. The undesired interaction among used substance can be found provided that the junction of leucocytes concentrates would be performed immediately following their obtaining from donor - before their freezing, i.e. while cellular components are still undamaged.

At the same time, existing cellular components in leucocytes homogenate are broken by repeated freezing and defrosting or by ultrasound destruction of cellular components or by combination of both the methods. During this method, the temperature is kept not to exceed + 37 °C. This temperature should never be higher by more than 20 °C.

### b) Dialysis:

Further treatment of leucocytes homogenate is performed in a closed system using the dialysing column. If a leucocytes homogenate has been frozen before further manufacturing, it must be defrosted in a water bath of not more than + 37 °C. This temperature can be lower. However the difference of the temperature by more than 30 °C brings an advantage.

Homogenate dialysis runs as a dialysis in closed system.

Dialysis is performed in dialysis unit obtained from apparatus with membrane keeping particles with relative molecular weight more than 10000. Dialysis is performed in lower temperature +2 °C - +8 °C. This temperature should not differ by more than 20 °C. The volume of concentrated leucocytes homogenate concentrate volume is ideally between 1,4 and 2,4 litres. It should not exceed tens of litres and be smaller than 0,2 l. The amount of water for injection that is needed for low molecule substances in range of 2 °C - 8 °C, is between 8 - 13 l. This amount of water can be different according to the used temperature however it should correspond to litres or hectolitres.

As an advantage, disintegrated leucocytes concentrate can circulate by force of pump through dialysing unit having a flow 1,2 - 2,0 l/hour and water for injections as a dialysing medium through the same dialysing unit by another circle having a flow 0,4 - 0,5 l/hour provided that the time for dialysis makes 24 h. ± 4 hour. If another circulation speeds are used, the time for dialysis must be adapted accordingly. Dialysis is completed as soon as absorbance level of dialysate drops under 0,6 in 260 nm. This value is recommended however it can be different.

Should an ultrafiltration be used instead of dialysis, the procedure is similar.

As an advantage, a commercial dialysis unit of dialysis machine is used that keeps particles of relative molecular weight higher than 10 000. A high standard of preparation is ensured.

Dialysis can be also replaced by ultrafiltration in this step.

### c) Concentration by means of lyophylisation:

In this step of manufacturing, first the obtained dialysate is distributed by 0,2 litre to suitable vessels (bottles) and then frozen under temperature less than -30°C. Further increase of concentration is performed by desiccation using a lyophylisation equipment for 20 - 30 hours in temperature up to + 30 °C until the half dry or semi liquid status is obtained. If a dialysate cannot be elaborated immediately, it must be kept in temperature not exceeding -17°C.

This manufacturing step is necessary especially for the fact that dialysate obtained contains too high part of water and must be carefully inspissated. There are different methods of inspissations. As far as transfer factor is concerned, a dialysate is inspissed by the lyophylisation (see above). The method is very careful, the manufactured product is again protected against undesired termic stress. The lyophylisation temperature should not exceed +30 °C. As an advantage, the temperature variation during the whole manufacturing step never exceeds 20 °C and filling-in volume is never bigger than 2 litre and smaller than 0,2 litre.

### d) Raw medication solution preparation:

The above mentioned lyophylisation leads to increased concentration of medicament. Following water dissolution should lead to such a concentration that is needed for treatment. Therefore concentrated product is qualitatively dissolved in water for injections immediately following lyophylisation so as one unit of product is contained in 1,5 ml. One unit represented by one injection therapeutic dose containing the amount of low molecule substances' that come from 2 x 10⁸ leucocytes.

Should a concentrate obtained by this way be not processed, it must be stored in glass bottles of 0,5 1, the temperature being at least -17 °C till the following work but not longer than 12 months.

Preparation of one batch takes usually not more than 3 months. The storage temperature should never exceed zero and shelf time never exceeds the given length.

One therapeutic dose should be obtained from the amount of leucocytes not smaller than1 million but not higher than one billion as an order (10⁹).

### e) Interoperation tests:

They are performed in dissolved concentrated product by means of absorbance evaluation in UV part of light spectrum in 260 nm and 280 nm. The ratio of values obtained in 260 a 280 nm is called "absorbance index". The value of this index must exceed 1,7. Raw transfer factor samples that do not meet this requirement, are excluded from further procedures.

### f) Homogenisation:

To get an injection substance, single batches of raw transfer factor are homogenised in stainless steel cauldron by steering following defrosting. The batch size of injection transfer factor must enable one lyophylisation cycle procedure in a given lyophylisation equipment.

### g) Pre-filtration:

The obtained solution must be filtered out immediately through the membrane of pore size 1,2 µm - 0,65 µm using an approx. 15 µm pre-filter.

### h) Ultrafiltration:

Following the pre-filtration, a filtrate is processed by ultrafiltration through membrane that makes a barrier to substances of relative molecular weight higher than 10 000.

### i) Sterilisation filtration:

The therapeutic utilisation expects a sterile product. Therefore ultrafiltrate is sterilised immediately via sterilisation filter for single use having pore size not more than 0,22 µm. Sterility can be achieved by different chemical or termical arrangements. The sterilisation of transfer factor is performed by sterilising filtration since possible the could be harmful for the product.

### j) Pasteurisation:

To be safe, the product must be inactivated. Transfer factor is inactivated by pasteurisation in +60°C for 10 hours in water bath. This procedure can ensure that final product is safe and, at the same time, not damaged thanks to its non-aggresivity. The process of pasteurisation must be recorded and evaluated continuously. The above mentioned course of inactivation can be performed also in another temperatures and timings. These deviations are possible however the safety of product for its use must be kept and at the same time it must not be damaged in terms of its therapeutic effectivity.

### k) Filling-in ampoules:

Following the pasteurisation, a sterile solution is portioned up. If needed, it can be stored in refrigerator (temperature being +2°C - +8°C) for not more than 3 days before this procedure. A fluid product is distributed from 2 1 glass bottles into 5 ml primary cover (e.g. ampoule) under sterile conditions in 1,5 ml parts using a suitable fillin-in equipment.

### l) Lyophylisation:

Primary packages with a filled in product are frozen on lyophylisation apparatus panels up to the temperature not higher than -30°C where they are lyophylisated up to the product temperature +30°C and residual humidity not more than 5%. The drying lasts 58 - 60 hours. The lyophylisation cycle lasts approx. 65 hours and can be longer with regards to the checked humidity of used material. The lyophylisation cycle can be terminated by stopping vacuum by gas nitrogen.

Primary packages with a dried product are closed using a suitable equipment by airproof closure immediately following the removal from lyophylisation apparatus. Lyophilised product is stored under the temperature of + 2 °C - + 8 °C, light protected.

Should storage conditions are kept, the shelf life of lyophylisated product is at least 2 years.

If a lyophylisated product does not suit in terms of appearance, humidity, current acidity, and sterility, it can be re-mastered by dissolving in water for injections and following the necessary changes, sterile filtration and tests can be filed in and lyophilised again.

Should effectivity tests not match, the product cannot be remastered and must be destroyed.

Inadequately finished product in ampoules that cannot be remastered, should be destroyed by crushing of primary packages. During the manufacturing process, a possibly contagious material is used. Therefore protective tools must be used during the work.

The final product is tested especially from the point of view of: appearance, airproof primary package, drying loss, solubility, current acidity, low molecular substances characteristics, pyrogenity tests, tests for non-harmfulness, sterility, effectivity.

During the manufacture process, the described temperature regimen must never be violated by more than 40 °C and the length of drying should not be shorter than 10 hours and not longer than 60 hours. The shelf life different from the above mentioned one is also possible provided that drug stability being certified.

The device can be used for commercial production of any transfer factor, both specific and non-specific, that is derived from peripheral blood leucocytes homogenate. It can be used in human medicament manufacturing as well as in veterinary medicament production.

In one distance of the present disclosure, The product can be used in situations where cellular immunity defect is proved and can be recommended in recurrent chronic infections, serious sepsis, eczema atopicum, dermatitis atopica, recurrent vaginal mycosis, psoriasis, chronic fatigue syndrome, serious problems of allergy or oncology origin.

The product can be used also as a prophylaxis (preoperative care in immunosupressed persons, during the treatment leading to undesired immunodeficiency).

Recommended dosage of transfer factor is 3 weekly doses. The forth one is given one month later in described situation in human and veterinary medicament.

All "%" refer to volume.

## Claims

1. Method of manufacturing "transfer factor" medicament having therapeutic effects based especially on donor immune system normalizing and containing complex and combination of biologically active substances having MW under 10000 with at least 200 biological activities, including transfer factors in donors' blood as are particularly precursors of maturation and activation of cells, **characterized by** involving following consequential steps in order:
1) leucocyte homogenate preparation
2) dialysis or ultrafiltration
3) concentration by means of lyophilization
4) preparation of raw medical solution
5) interoperation tests
6) homogenization
7) pre-filtration
8) ultrafiltration
9) sterile filtration
10) pasteurization
11) filling in ampoules and
12) lyophilization.

2. Method of manufacturing "transfer factor" medicament acc. to claim no. 1, wherein the leucocyte homogenate is prepared by freezing, thawing, by ultrasound or combination thereof, of human leucocyte concentrate obtained from pooled individual donors.

3. Method of manufacturing "transfer factor" medicament acc. to claims no. 1 and 2, wherein the temperature of the manufacture process at every consequential manufacturing step is regulated in order not to exceed 37°C except for pasteurization.

4. Method of manufacturing "transfer factor" medicament acc. to claims no. 1 - 3, wherein the manufacturing step "dialysis" is carried out with use of commercial dialysis unit keeping substances of MW higher than 10000.

5. Method of manufacturing "transfer factor" medicament acc. to claims no. 1 - 3, wherein the manufacturing step "dialysis" may be advantageously replaced by ultrafiltration.

6. Method of manufacturing "transfer factor" medicament acc. to claims no. 1 - 4, wherein obtained dialysate is filled per 0.2 litre before freeze drying concentration and it is freezed at -30°C and lower temperature, then it is concentrated in freeze dryer for 20-30 hours at the temperature of material of max. 30°C until the material is half dry or semi-liquid.

7. Method of manufacturing "transfer factor" medicament acc. to claims no. 1 - 4, wherein obtained dialysate is filled per 0.2 litre and it is stored at - 17°C and lower temperature up to next processing.

8. Method of manufacturing "transfer factor" medicament acc. to claims no. 1 - 5 and 7, wherein the bulk solution is prepared by quantitative dissolution of concentrate in water for injection "WFI" immediately after concentration, so that 1.5 ml of preparation contains one dose, which is one terapeutical dose containing the amount of substances of low MW obtained by manufacturing of 2x10⁸ of leucocytes.

9. Method of manufacturing "transfer factor" medicament acc. to claim no. 8, wherein the bulk solution is not processed immediately after dissolution in WFI, but it is stored in bottles of 0.5 litre volume at -17°C and lower temperature for max. 12 months up to next processing.

10. Method of manufacturing "transfer factor" medicament acc. to claims no. 1 - 9, wherein one therapeutical dose of the crude medicament is obtained from leucocytes, the quantity of which ranging from 1x10⁶ to 1x10⁹.

11. Method of manufacturing "transfer factor" medicament acc. to claims 1 to 10, wherein the sterilizing filtration is made using a disposable filter with a pore diameter of max. 0.22 µm and the sterility of the medicament is assured by aseptic manufacturing without adding antimicrobial preservative.

12. Method of manufacturing "transfer factor" medicament acc. to claims no. 1 - 11, wherein the heat inactivation by pasteurization in water bath at 60°C for 10 hours is undertaken.

13. Method of manufacturing "transfer factor" medicament acc. to claims no. 1 - 12, wherein the drying period of lyofilization is not shorter than 10 hours and longer than 60 hours.

## Patentansprüche

1. Art der Herstellung des Medikaments "Tansfer Faktor" mit Heilwirkungen, die insbesondere in der Aufbereitung der Funktionen des Immunsystems des Spenders beruhen und einen Komplex und eine Kombination biologisch aktiver niedrigmolekularer Stoffe mit einer relativen Molekülmasse unter 10 000 mit annähernd 200 verschiedenen biologischen Aktivitäten enthalten, die im Blut der Spender enthaltende Übertragungsfaktoren umfassen, wie es insbesondere Präkursoren des Ausreifens und der Aktivierung von Zellen sind, die **dadurch gekennzeichnet ist, dass** die Art folgende schrittweise Schritte umfasst:
1) Vorbereitung des Leukozyten-Homogenats;
2) Dialyse oder Ultrafiltration;
3) Konzentration durch Lyophilisation;
4) Vorbereitung der Lösung ces rohen Medikaments;
5) zwischenoperative Prüfungen;
6) Homogenisierung;
7) Vorfiltration;
8) Ultrafiltration;
9) Sterilisierende Filtration;
10) Wärmeinaktivierung;
11) Abfüllen in Ampullen und
12) Lyophilisation.

2. Art der Herstellung des Medikaments "Transfer Faktor" gemäß Anspruch 1, die **dadurch gekennzeichnet ist, dass** das Leukozyten-Homogenat durch Einwirken auf das Leukozyten-Konzentrat, das von den verbundenen einzelnen Spendern gewcnnen wird, vorbereitet wird, wobei dieses Einwirken das Einfrieren, Auftauen, das Einwirken von Ultraschall oder deren Kombination sind.

3. Art der Herstellung des Medikaments "Transfer Faktor" gemäß den Ansprüchen 1 und 2, die **dadurch gekennzeichnet ist, dass** die Temperatur bei allen allmählichen Produktionsschritten außer der Wärmeinaktivierung so reguliert wird, dass sie + 37 C nicht übersteigt.

4. Art der Herstellung des Medikaments "Transfer Faktor" gemäß den Ansprüchen 1 bis 3, die **dadurch gekennzeichnet ist, dass** der Schritt der Dialyse unter Nutzung einer kommerzielien Dialyseeinheit erfolgt, um Teilchen mit einer relativen Molekülmasse über 10000 zurückzuhalten.

5. Art der Herstellung des Medikaments "Transfer Faktor" gemäß den Ansprüchen 1 bis 3, die **dadurch gekennzeichnet ist, dass** der Schritt Dialyse vorteilhaft durch Ultrafiltration ersetzt wird.

6. Art der Herstellung des Medikaments "Transfer Faktor" gemäß den Ansprüchen 1 bis 4, die **dadurch gekennzeichnet ist, dass** das gewonnene Dialyseprodukt vor der Konzentration dur Lyophilisation zu je 0,2 Litern abgefüllt und bei einer Temperatur von mindestens -30°C eingefroren wird, weiterhin wird es durch Trocknen in eine Lyophilisationsanlage über einen Zeitraum von 20 - 30 Stunden bei einer Temperatur des Materials bis + 30°C bis zum halbtrockenen oder halbflüssigen Zustand konzentriert.

7. Art der Herstellung des Medikamenys "Transfer Faktor" gemäß den Ansprüchen 1 bis 4, die **dadurch gekennzeichnet ist, dass** das gewonnene Dialyseprodukt zu je 0,2 Litern abgefüllt und bei einer Temperatur von - 17°C und tiefer bis zur weiteren Verarbeitung aufbewahrt wird.

8. Art der Herstellung des Medikarments "Transfer Faktor" gemäß den Ansprüchen 1 bis 5 und 7, die **dadurch gekennzeichnet ist, dass** die Lösung des rohen Medikaments unmittelbar durch quantitative Auflösung des Produkts der Lyophilisation in Wasser zur Injektion so vorbereitet wird, dass eine Einheit des Mittels in einer Menge von 1,5 ml enthalten ist, wobei unter einer Einheit eine therapeutische Injectionsdosis verstanden wird, und darin ist eine solche Menge niedrigmole kularer Stoffe enthalten, wie sie durch Verarbeitung von annähernd 2 x 10⁸ Leukozyten gewonnen wird.

9. Art der Herstellung des Medikaments "Transfer Faktor" gemäß dem Anspruch 8, die **dadurch gekennzeichnet ist, dass** das Produkt nach der Auflösung des Lyophilisat nicht unmittelbar weiter zur Injektion verarbeitet wird, sondern in Flaschen mit einem Volumen von 0,5 l bei einer Temperatur von min. -17°C bis zum Zeitpunkt der weiteren Verarbeitung, höchstens jedoch für einen Zeitraum von 12 Monaten, aufbewahrt wird.

10. Art der Herstellung des Medikaments "Transfer Faktor" gemäß den Ansprüchen 1 bis 9, die **dadurch gekennzeichnet ist, dass** eine therapeutische Dosis der Lösung des rohen Medikamentes aus einer Leukozytenmenge in der Spanne von 1x10⁶ bis 1x10⁹ gewonnen wird.

11. Art der Herstellung des Medikaments "Transfer Faktor" gemäß den Ansprüchen 1 bis 10, die **dadurch gekennzeichnet ist, dass** die sterilisierende Filtration über einen Einmalfilter mit einer Porengröße von maximal 0,22 µm erfolgt und die Sterilität des Medikaments durch aseptische Zubereitung sichergestellt wird, ohne dass antimikrobielle Konservierungsmittel zugesetzt werden müssen.

12. Art der Herstellung des Medikaments "Transfer Faktor" gemäß den Ansprüchen 1 bis 11, die **dadurch gekennzeichnet ist, dass** die Wärmeinaktivierung durch Pasteurisierung bei der Temperatur von +60°C über einen Zeitraum von 10 Stunden im Wasserbad erfolgt.

13. Art der Herstellung des Medikaments "Transfer Faktor" gemäß den Ansprüchen 1 bis 12, die **dadurch gekennzeichnet ist, dass** die Trocknungsdauer bei der Lyophilisation nicht kürzer als 10 Stunden und nicht länger als 60 Stunden ist.

## Revendications

1. Le procédé de préparation du produit médicamenteux « transfer faktor » ayant des effets thérapeutiques basés principalement sur la modification des fonctions du système immunitaire du donneur et contenant un ensemble et une combinaison de substances de faible poids moléculaire biologiquement actives qui ont une masse moléculaire relative inférieure à 10 000, présentent environ deux cents activités biologiques différentes et englobent des facteurs de transmission contenus dans le sang des donneurs, comme notamment des précurseurs de maturation et d'activation cellulaires, est **caractérisé en ce qu'**il comporte les étapes successives suivantes :
1) préparation d'homogénat leucocytaire
2) dialyse ou ultrafiltration
3) concentration par lyophilisation
4) préparation de solution de médicament brut
5) tests intermédiaires
6) homogénéisation
7) préfiltration
8) ultrafiltration
9) filtration stérilisante
10) inactivation thermique
11) remplissage des ampoules
12) lyophilisation.

2. Le procédé de préparation du produit médicamenteux « transfer faktor » selon la revendication 1 caractérisé en ce qui l'homogénat leucocytaire est préparé en agissant sur le concentré leucocytaire obtenu à partir de différents donneurs associés et ce traitement consiste en une congélation, une décongélation et une action des ultrasons ou leur combinaison.

3. Le procédé de préparation du produit médicamenteux « transfer faktor » selon les revendications 1 et 2 **caractérisé en ce que** la température est régulée afin de ne pas dépasser +37°C pendant toutes les étapes successives de préparation, sauf pendant l'inactivation thermique.

4. Le procédé de préparation du produit médicamenteux « transfer faktor » selon les revendications 1 à 3 caractétisé en ce que l'étape de dialyse s'effectue à l'aide de l'unité de dialyse commerciale ayant pour but la rétention de particules qui ont une masse moléculaire relative supérieure à 10000.

5. Le procédé de préparation du produit médicamenteux « transfer faktor » selon les revendications 1 à 3 **caractérisé en ce que** l'étape de dialyse est remplacée de manière avantageuse par l'ultrafiltration.

6. Le procédé de préparation du produit médicamenteux « transfer faktor » selon les revendications 1 à 4 **caractérisé en ce que** le dialysat obtenu est divisé en doses de C,2 litres et congelé à une température minimale de -30°C avant la concentration par lyophilisation, puis concentré par séchage dans un appareil de lyophilisation pendant 20-30 heures à une température de matériel inférieure à +30 °C jusqu'à un état demi-sec, voire demi-liquide.

7. Le procécé de préparation ou produit médicamenteux « transfer faktor » selon les revendications 1 à 4 **caractérisé en ce que** le dialysat obtenu est divisé en doses de 0,2 litres et conservé à une température de -17 °C ou inférieure avant la transformation suivante.

8. Le procédé de préparation du produit médicamenteux « transfer faktor » selon les revendications 1 à et 7 **caractérisé en ce que** la solution de produit médicamenteux brut est préparée par une dissolution quantitative immédiate du produit de lyophilisation dans l'eau destinée pour l'injection de telle sorte qu'une unité de préparation soit contenue dans une quantité de 1,5 ml, sachant qu'une unité représente une dose thérapeutique d'injection contenant la quantité de substances à faible poids moléculaires obtenues par la transformation d'environ x 10⁸ leucocytes.

9. Le procédé de préparation du produit médicamenteux « transfer faktor » selon la revendication 8 **caractérisé en ce que** le produit n'est pas immédiatement transformé en injections après la dissolution de lyophilisat mais il est stocké dans des bouteilles d'un volume de 0,5 1 à une température minimale de -17°C en attendant les transformations suivantes et sans dépasser une durée de 12 mois.

10. Le procédé de préparation du produit médicamenteux « transfer faktor » selon les revendications 1 à 9 **caractérisé en ce qu'**une dose thérapeutique de solution de produit médicamenteux brut est obtenue à partir d'un nombre de leucocytes allant de 1 x 10⁶ à 1 x 10⁹.

11. Le procédé de préparation du produit médicamenteux « transfer faktor » selon les revendications 1 à 10 **caractérisé en ce que** la filtration stérilisante est effectuée à l'aide d'un filtre à usage unique avec des pores d'une dimension maximale de 0,22 µm et la stérilité du produit médicamenteux est assurée par une préparaticn aseptique qui ne nécessite pas l'ajout de substances antimicrobienne de conservation.

12. Le procédé de préparation du produit médicamenteux « transfer faktor » selon les revendications 1 à 11 **caractérisé en ce que** l'inactivation thermique par pasteurisation est effectuée au bain-marie à une température de +60 °C pendant une durée de 10 heures.

13. Le procédé de préparation du produit médicamenteux « transfer faktor » selon les revendications 1 à 12 **caractérisé en ce que** la durée de séchage lors de la lyophilisation est supérieure à 10 heures et inférieure à 60 heures.
